# EUROPEAN PATENT APPLICATION

(11) **EP 4 744 652 A2**
(43) Date of publication of application: **20.05.2026**
(21) Application number: 26155177.4
(22) Date of filing: 27.01.2023
(51) Int. Cl.: A61K 9/50

(54) **A METHOD OF FORMING A COMPOSITION COMPRISING A PROBIOTIC MICRO-ENCAPSULATED IN A DENATURED PLANT PROTEIN MATRIX**

(30) Priority: 27.01.2022 GB 202201083
(62) Divisional of application: 23702555.6
(71) Applicant: Anabio Technologies Limited, Dublin, D02 RW27 (IE)
(72) Inventor: Bleiel, Sinead, Dublin, D02 RW27 (IE); Severic Balunovic, Maja, Cork, T12 TX06 (IE)
(74) Representative: Purdylucey Intellectual Property

(57) **Abstract**

A method of forming a composition comprising a probiotic encapsulated in a denatured plant protein matrix, comprises the steps of preparing a protein suspension comprising denatured plant protein, preparing a suspension of hydrated probiotic, combining the protein suspension and the suspension of hydrated probiotic to encapsulate the probiotic in a denatured pea protein matrix, and polymerising the denatured plant protein matrix with a calcium salt. The combining step may comprise extruding the protein suspension and the suspension of hydrated probiotic to form microdroplets, in which the polymerisation step comprises curing the extruded microdroplets in a curing bath comprising a calcium citrate buffer having a pH of 5 to 6.5 and a molarity of 0.05 to 0.15 M. The combining step may also comprise mixing the protein suspension and probiotic suspension to form a mixture, adding a calcium salt buffer to the mixture to gel the mixture, and drying the gelled mixture by freeze-drying or vacuum drying.

## Description

### Field of the Invention

The present invention relates to a method of forming a composition comprising a probiotic micro-encapsulated in a denatured plant protein matrix. The invention also relates to method of forming a denatured plant protein suspension. The invention also provides products and compositions formed according to the methods of the invention.

### Background to the Invention

Manufacturing microencapsulated active agents such as probiotic bacteria that are robust, protect the encapsulated probiotic bacteria, and are storage stable, requires the use of protein suspensions that have a high solids content and a high proportion of the protein in solution. WO2008/056344 describes the encapsulation of probiotic bacteria in a denatured whey protein matrix using a cold gelation process in which the probiotic bacteria are mixed with a suspension of denatured whey, extruded into microdroplets, which are cured in an acidic bath at a pH of 4.6. Depending on the probiotic, there is often an adverse reaction when probiotics are added to whey protein via the cold gelation method, which leads to solidification and loss of cell counts. Furthermore, the pH required to create cold gelated whey protein is often deleterious for probiotic survival e.g. pH 4.6.

It is an objective of the invention to overcome at least one of the above-referenced problems.

### Summary of the Invention

The objective is met by the provision of a method for encapsulating probiotics that employs plant protein as the encapsulating matrix. The Applicant has discovered that using a plant protein such as denatured pea protein and denatured mung bean protein as a micro-encapsulating matrix for probiotic bacteria results in a greater number of bacteria surviving the encapsulation process, for example >75-80 % survival of cells compared to 40 - 50% with dairy based encapsulation systems, such as WPI or MPC. Without being bound by theory, it is believed that the increase in survival is due to the plant protein polymerising at a pH of 6-7 as opposed to a pH of 4.6 for whey protein. A further advantage of the invention is that the use of plant protein as an encapsulation matrix makes the product suitable for use by vegans and vegetarians. The disclosure describes a number of methods of encapsulating probiotics with denatured pea protein and denatured mung bean protein (as an example of a plant protein), all of which employ a calcium salt chelating agent that is capable of polymerising denatured plant protein at a weakly acidic pH, a pH which does not have a detrimental effect on probiotics survival or metabolism. Examples provided herein show encapsulated probiotics made by extrusion into a gelling bath, and by gel immobilisation and drying (for example freeze or vacuum drying). The invention also describes a method of making a suspension of plant protein (generally denatured pea protein or denatured mung bean protein) that is obtained by hydration of the protein at pH 7-8, resting the protein suspension, and a two-stage heating step typically carried out under elevated pressure. Embodiments of the process has been found to produce suspensions of plant protein that are high in solids (e.g. up to 15% solids), have a high proportion of soluble protein (e.g. 90% of more), and do not have a highly acidic or alkaline pH, which makes the suspension ideal for micro-encapsulating pH sensitive agents such as probiotics. Furthermore, an alternative heat treatment (ultra high temperature (UHT) denaturation) is employed to denature the plant protein which results in a more efficient encapsulation process due to the reduction in viscosity of the protein solution. The method of the present disclosure may also be applied to encapsulate other active agents such as botanical extracts, vitamins, minerals, marine bioactives and amino acids

In a first aspect, the invention provides a method of forming a microparticle comprising an active agent such as a probiotic encapsulated in a denatured plant protein matrix, comprising the steps of
preparing a protein suspension comprising denatured plant protein;
combining the protein suspension and active agent to form a mixture;
treating the mixture to form a microparticle comprising active agent encapsulated in a denatured plant protein matrix, in which the treating step comprises polymerising the denatured plant protein matrix with a calcium salt or spray englobing on a fluidised bed dryer; and
drying the microparticle.

In any embodiment, the method includes a step of preparing a suspension of hydrated probiotic, wherein the combining step comprises combining the protein suspension with the suspension of hydrated probiotic.

In any embodiment, the plant protein is or comprises a plant protein isolate.

In any embodiment, the plant protein is or comprises pea protein or mung protein.

In any embodiment, a simple carbohydrate is added to the protein suspension. Examples include monosaccharides and/or disaccharides. A simple carbohydrate is added to the suspension pre-encapsulation to create a charged slurry to enhance compatibility of the denatured plant protein suspension with the probiotic cells for stable equilibrium of cells in protein matrix during encapsulation and create a charged slurry that interacts with the respective charged cell membrane of the probiotic. The simple carbohydrate also helps generate an instantaneous chelation / polymerisation due to the interaction between charged simple carbohydrate and denatured plant protein after heat treatment. This charge interaction creates a robust lattice for entrapment and encapsulation of sensitive bacteria.

In any embodiment, the treating step comprises extruding the protein suspension and the suspension of active agent to form microdroplets, in which the polymerisation step comprises curing the extruded microdroplets in a curing bath comprising a chelating agent such as a calcium salt to form microcapsules.

In any embodiment, the curing bath comprises a calcium citrate buffer.

In any embodiment, the curing bath comprises a calcium citrate buffer having a pH of 5 to 6.5

In any embodiment, the curing bath comprises a calcium citrate buffer having a molarity of 0.05 to 0.15 M.

In any embodiment, the curing bath comprises a calcium citrate buffer having a pH of 5.2 to 6.1.

In any embodiment, the curing bath comprises a calcium citrate buffer having a molarity of about 0.1 M.

In any embodiment, the curing bath comprises a calcium chloride buffer.

In any embodiment, the curing bath comprises a calcium chloride buffer having a pH of 4.3 to 5.

In any embodiment, the curing bath comprises a calcium chloride buffer having a molarity of 0.05 to 0.15 M, or about 01.M.

In any embodiment, the curing bath comprises a surfactant, especially a non-ionic surfactant, especially a polysorbate-type non-ionic surfactant. In any embodiment, the curing bath comprises 0.01 to 0.1, especially about 0.05%, surfactant.

In any embodiment, the curing bath comprises chitosan In any embodiment, the curing bath comprises 0.01 to 0.1, especially about 0.05%, chitosan.

In any embodiment, the treating step comprises adding a calcium salt buffer to gel the mixture, drying the gelled mixture by freeze-drying or vacuum drying, and size reducing the dried gelled mixture to provide the microparticles.

In any embodiment, the calcium salt buffer comprises a calcium citrate buffer having a pH of 5 to 6.5.

In any embodiment, the calcium salt buffer comprises a calcium citrate buffer having a molarity of 0.05 to 0.15 M.

In any embodiment, the calcium salt buffer is added to the mixture at a volumetric ratio of 1:100 to 1:300.

In any embodiment, the protein suspension comprises 10 to 15%, 11 to 15%, 12 to 15%, 13 to 15% or 14 to 15% denatured plant protein (by weight).

In any embodiment, at least 90%, 91%, 92%, 93%, 94% or 95% of the protein in the protein suspension is solubilised.

In any embodiment, the protein suspension comprises 0.5 to 12.0%, 1.0 to 6.0%, 1.0-3.0% or about 2% simple sugar (w/v)
In any embodiment, the combining step comprises combining the protein suspension and the suspension of active agent at a denatured plant protein to active agent dry weight ratio of 1:5 to 1:25 or 1:8 to 1:20.

In any embodiment, the active agent is a probiotic, and the suspension of hydrated probiotic is suspended in a 0.05 to 0.15 M, 0.08 to 0.12 M, or about 0.1 M phosphate buffer.

In any embodiment, the simple carbohydrate comprises maltodextrin and glucose. GLUCIDEX is a commercial product containing maltodextrin and glucose.

In any embodiment, the method comprises forming the microparticles by spray englobing in which the combining and treating steps are performed on a fluidised bed dryer.

In any embodiment, the method comprises adding a carrier material and an active agent to a bed of a fluidised bed dryer, fluidising the carrier material and active agent, spraying a first coating material onto the fluidised carrier material and active agent to produce a microparticles having an active agent and carrier contained within a shell of first coating material, and drying the microparticles.

In any embodiment, a simple sugar is sprayed into the fluidised bed dryer prior to the first coating material being sprayed into the fluidised bed dryer, wherein the simple sugar produces granules and the first coating material coats the granules.

In any embodiment, the first coating material is selected from denatured plant protein, oil, and a simple sugar such as maltodextrin.

In any embodiment, the method comprises spraying a second coating material on the shell of first coating material, wherein at least one of the first and second coating materials is denatured plant protein..

In any embodiment, the method comprises spraying a chelating salt on the denatured plant protein coating.

The following microparticles are envisaged:
Microparticle 1
   Core of active agent (e.g. probiotic) and carrier material
   Coating of polymerised denatured plant protein
Microparticle 2
   Core of active agent (e.g. probiotic), simple sugar and carrier material
   Coating of non-polymerised dried denatured plant protein
Microparticle 3
   Core of active agent (e.g. probiotic) and carrier material
   Inner coating of polymerised denatured plant protein
   Outer coating of non-polymerised dried denatured plant protein
   Intermediate coating of oil.
Microparticle 4
   Core of active agent (e.g. probiotic) and carrier material
   Inner coating of polymerised denatured plant protein
   Outer coating of polymerised denatured plant protein
   First intermediate coating of oil.
   Second intermediate coating of non-polymerised dried denatured plant protein

In one embodiment, the spray englobing comprises the steps of:
(a) adding a carrier material (e.g. a native protein), an active agent (e.g. a probiotic), and optionally a simple sugar, to a bed of a fluidised bed drying chamber;
(b) fluidising and heating the carrier material and active to form a first fluidised powder;
(c) spraying the denatured protein suspension onto the fluidised bed at elevated pressure (e.g. 2-4.5 Bar) to provide a second fluidised powder;
(d) drying the second fluidised powder on the fluidised bed to reduce the moisture content of the second fluidised powder (e.g. reduce moisture content by at least 30%, 40% or 50%);
(e) spraying an englobing component on to the second fluidised powder to form a third fluidised powder, in which the englobing component is selected from a chelating salt (e.g. a calcium salt or a magnesium salt); an edible oil, and a simple sugar;
(f) drying the third fluidised powder on the fluidised bed to further reduce the moisture content of the fluidised powder (e.g. further reduce moisture content by at least 10%, 20% or 25%);
(g) spraying the denatured protein suspension onto the fluidised bed at elevated pressure (e.g. 2-4.5 Bar) to provide a fourth fluidised powder comprising microparticles; and
optionally, further drying the fourth fluidised powder, typically to a moisture content of less than 10%, 8%, or 5%.

In any embodiment, the process comprises at least 2 or 3 rounds of steps (f) and (g).

In any embodiment, when the englobing component is an edible oil, the process includes an additional step of spraying a chelating salt onto the third fluidised powder prior to the second drying step. The method may comprise at least 2 or 3 rounds of spraying the edible oil and then spraying the chelating salt.

Generally, the spraying steps referenced above are top spraying, e.g. the component is sprayed from above the fluidised bed.

In any embodiment, the method comprises the following additional steps:
(h) bottom spraying denatured protein suspension onto the fourth fluidised mixture to form a fifth fluidised powder;
(i) drying the fifth fluidised powder on the fluidised bed to further reduce the moisture content of the fluidised powder (e.g. further reduce moisture content by at least 10%, 20% or 25%);
(j) bottom spraying an englobing component on to the fifth fluidised powder to form a sixth fluidised powder, in which the englobing component is selected from a chelating salt (e.g. a calcium salt or a magnesium salt) and an edible oil;
(k) drying the sixth fluidised powder on the fluidised bed to further reduce the moisture content of the fluidised powder (e.g. further reduce moisture content by at least 10%, 20% or 25%); and
(l) optionally, further drying the fourth fluidised powder, typically to a moisture content of less than 10%, 8%, or 5%.

In any embodiment, the process comprises at least 2 or 3 rounds of steps (j) and (k).

In any embodiment, when the englobing component of step (j) is an edible oil, the process includes an additional step of spraying a chelating salt onto the sixth fluidised powder prior to step (k). The method may comprise at least 2 or 3 rounds of spraying the edible oil and then spraying the chelating salt.

In any embodiment, the fluidised bed is fluidised with an airflow of 110 to 300 m³/hour.

In any embodiment, the fluidised bed chamber is heated during all or part of the method, typically to 35 to 45 °C.

In any embodiment, the protein solution is sprayed into the fluidised bed at an elevated spray nozzle pressure, e.g. 2 to 4.5 Bar.

In any embodiment, the simple sugar is added to the second fluidised powder in an amount of 10-20% total dry solids.

In any embodiment, the simple sugar is or comprises maltodextrin.

In any embodiment, the chelating salt is added as a 0.2 - 0.4 M solution.

In any embodiment, the edible oil is added to the second fluidised powder in an amount of 5-15 % total dry solids.

In any embodiment, step (a) comprises adding the carrier material and the active agent to the bed of a fluidised bed drying chamber in a weight ratio of 1:5 to 5:1..

The carrier material is generally native protein, but may also be a carbohydrate such as maltodextrin or starch, a prebiotic fibre powder or any colloid.

In another embodiment, the spray englobing comprises the steps of:
fluidising a carrier material such as native protein, an active agent (such as a probiotic), and a chelating salt on a fluidised bed of a fluidised bed drying chamber;
spraying an edible oil into the fluidised bed drying chamber;
spraying a suspension of denatured protein into the fluidised bed drying chamber,
whereby the chelating salt reacts with the denatured plant protein to polymerise the protein and form agglomerated microparticles having a polymerised denatured protein coat and core comprising active agent and carrier material.

In any embodiment, the carrier material is added first to the fluidised bed, and the active agent and chelating salt are added to the fluidised bed after the carrier material.

In any embodiment, the carrier material is fluidised at elevated temperature (e.g. 30-40°C) prior to the addition of the active agent and chelating salt.

In any embodiment, the carrier material, active agent, and chelating salt are fluidised at elevated temperature (e.g. 30-40°C) prior to the addition of the edible oil to form a fluidised mixture.

In any embodiment, a simple sugar is sprayed onto the fluidised mixture prior to the edible oil to promote granulation of the mixture.

In any embodiment, the simple sugar is sprayed into the fluidised bed chamber at high flow rate (e.g. 23 to 30 RPM), low airflow (e.g. >150 RMP), and low spray nozzle pressure (e.g. <1.5 Bar).

In any embodiment, the oil is sprayed into the fluidised bed chamber at low flow rate (e.g. 18-22 RPM), high airflow (e.g. >150 RMP), and low spray nozzle pressure (e.g. <1.5 Bar).

In any embodiment, the suspension of denatured plant protein is sprayed into the fluidised bed drying chamber at high flow rate (e.g. 23-30 RPM), high airflow (e.g. >150 RMP), and high spray nozzle pressure (e.g. >2.5 Bar).

In any embodiment, the active agent is added in the form of an oil-in-water nanoemulsion, in which the active agent is contained in the oil phase of the oil-in-water nanoemulsion. This is particularly relevant when the active agent is hydrophobic.

In any embodiment, the oil-in-water nanoemulsion is an oil-in-water nanoemulsion of the invention.

In any embodiment, the carrier material is native protein typically having a moisture content of about or less than 5%, 4%, 3%, or 2%.

In any embodiment, plant the oil is an edible oil, such as a high oleic oil.

In any embodiment, the bed is fluidised with air at 30 to 40 °C and a high airflow rate (approximately 30 to 55 RPM).

In any embodiment, the native protein and denatured protein are plant protein, for example pea or mung bean protein.

In any embodiment, the native protein and denatured protein are protein isolates.

In any embodiment, the suspension of denatured plant protein is a denatured plant protein suspension of the invention.

In any embodiment, the agglomerated microparticles are gastric resistant and ileal sensitive. This means that the microparticles can pass through a human stomach intact and break up in the ileum releasing the active agent payload.

In any embodiment, the protein suspension is formed by a process comprising the steps of:
hydrating plant protein in an aqueous solvent at pH 7 to 8 with agitation and optionally heating;
resting the aqueous suspension of plant protein;
adjusting the pH of the aqueous suspension to 7 to 8 if necessary;
heating the rested aqueous suspension optionally under pressure; and
cooling the heated aqueous suspension.

In any embodiment, the aqueous suspension of plant protein is rested for at least 30, 60 or 90 minutes.

In any embodiment, the hydration step is carried out at a temperature of 25 to 40 °C, 30 to 40 °C, and ideally about 35 °C.

In any embodiment, the heating step comprises:
pre-heating the rested aqueous suspension under pressure at a first heating temperature of 60 to 70 °C (e.g. about 65 °C) for a first heating time (e.g 5 seconds to 15 minutes); and
heating the pre-heated aqueous suspension under pressure at a second heating temperature of 90 to 100 °C (e.g. about 95 °C) for a second heating time (2 seconds to 3 minutes).

In any embodiment, the heating step comprises heating the suspension of plant protein to achieve a heat treatment of F0=3. This results in denaturation of the plant protein (e.g. to at least 80% denaturation) and kills bacterial spores in the suspension. The heat treatment also lowers the viscosity of the protein suspension making it easier to process. The term "F0 " is defined as the number of equivalent minutes of steam sterilization at 250°F (121°C) delivered to a load (product).

The ultra-high temperature denaturation process serves three purposes when denaturing the plant protein: (a) it kills any bacterial spores in the protein suspension rendering the product of food-grade quality and ensuring they are safe for human consumption, (b) denaturation at this high of a temperature is necessary to provide the relevant functionality to polymerise plant proteins, and (c) it also generates a viscosity that permits for better flow rates during the encapsulation process resulting in higher efficiency in the encapsulation process and higher throughput during encapsulation. The ultra-high temperature denaturation process has been found to reduce the viscosity of the plant protein suspension from 300 - 550cP at 21+/-1 °C to 50 - 120cP at 21 +/-1 °C. This process also helps ensure that if the microparticles of the invention are fortified into a beverage, it can be confirmed that the encapsulated material is clean of any contaminants and acceptable for commercial use and human consumption.

In any embodiment, the heating step comprises ultra-heat treatment (UHT) of the suspension of plant protein.

In any embodiment, the heat step comprises heating the suspension to about 138.5°C x about 3-4 secs.

In any embodiment, the aqueous solvent has a pH of about pH 7.5 (e.g. 7.3 to 7.7).

In any embodiment, the heating step is performed at a pressure of 1.3 to 1.6 Bar.

In any embodiment, the pea protein is or comprises pea protein isolate or mung bean protein isolate.

In any embodiment, the cooled protein suspension is allowed to settle for at least 2 hours at room temperature.

In any embodiment, the method includes a step of adding a simple carbohydrate to the protein suspension to provide a protein suspension typically comprising 0.5 to 5.0%, 1-3% or about 2% simple carbohydrate (w/v).

In any embodiment, the method has a % probiotic survival of greater than 50%, 55%, 60%, 65%, 70% or 75%. % probiotic survival as applied to the method of the invention means the % of the probiotic bacteria that survive the encapsulation process. A method of calculating % probiotic survival is provided below.

In another aspect, the invention provides a composition comprising an active agent such as a probiotic encapsulated in a denatured plant protein matrix, formed according to the method of the invention. The composition is generally particulate, for example microparticulate, and the microparticles may be granulates..

In any embodiment, the microparticles may have an average dimension of (in microns, µm) 50-700, 200-700, 300-700, 400-700, 500-600, 50-400, 50-300, 50-200, 50-150, 50-100, 20-100, 20-50, 100-500, 100-400, 100-300, or 100-200. When formed by extrusion into a bath, the microparticles typically have an average dimension of 5000 to 600 µm. When a single nozzle is employed, the microparticles have a continuous denatured protein matrix and probiotic bacteria distributed throughout the matrix (these are term microbeads). When a concentric nozzle is employed, the microparticles have a core-shell morphology, with a shell comprising denatured protein and a core comprising probiotic bacteria (these are term microcapsules). When formed by gel immobilisation, the microparticle typically have an average dimension of 50 to 150 µm. When formed by spray englobing, the microparticles typically have an average dimension Dv of 50 to 500, 100-500, 200-500, 300-400 µm.

In any embodiment, the microparticles are gastric resistant and ileal sensitive. This means that the microparticles can pass through a human stomach intact and break up in the ileum releasing the probiotic payload for subsequent colonisation.

In another aspect, the invention provides a method of forming a denatured plant protein suspension comprising the steps of:
hydrating a plant protein in an aqueous solvent at pH 6 to 8 or 7 to 8 with agitation and optional heating;
resting the aqueous suspension of plant protein;
adjusting the pH of the aqueous suspension to 6 to 8 or 7 to 8 if necessary;
heating the rested aqueous suspension ; and
cooling the heated aqueous suspension.

In any embodiment, the rested aqueous suspension is heated to achieve a heat treatment of F0=3.

In any embodiment, the rested aqueous suspension is subjected to ultra-heat treatment (UHT).

In any embodiment, the rested aqueous suspension is heated to about 138.5°C x about 3-4 secs.

In any embodiment, the aqueous suspension of plant protein is rested for at least 30, 60 or 90 minutes.

In any embodiment, the hydration step is carried out at a temperature of 25 to 40°C, 30 to 40°C, and ideally about 35°C.

In any embodiment, the heating step comprises:
pre-heating the rested aqueous suspension under pressure at a first heating temperature of 60 to 70 °C (e.g. about 65 °C) for a first heating time (e.g. 5 seconds to 15 minutes); and
heating the pre-heated aqueous suspension under pressure at a second heating temperature of 90 to 100 °C (e.g. about 95 °C) for a second heating time (2 seconds to 3 minutes).

In any embodiment, the aqueous solvent has a pH of about pH 7.5 (e.g. 7.3 to 7.7).

In any embodiment, the heating step is performed at a pressure of 1.3 to 1.6 Bar.

In any embodiment, the plant protein is a plant protein isolate.

In any embodiment, the plant protein is pea protein.

In any embodiment, the plant protein is mung bean protein.

In any embodiment, the cooled protein suspension is allowed to settle for at least 1, 2, 3, 4 or 5 hours at room temperature.

In any embodiment, the method includes a step of adding a simple carbohydrate to the protein suspension to provide a protein suspension typically comprising 0.5 to 5.0%, 1-3% or about 2% simple carbohydrate (w/v).

In any embodiment, the native protein and denatured protein are plant protein, for example pea or mung bean protein. In any embodiment, the native protein and denatured protein are from the same source (e.g. both pea protein). In any embodiment, the native protein and denatured protein are protein isolates.

In any embodiment, the suspension of denatured plant protein is a denatured plant protein suspension of the invention.

In any embodiment, the denatured plant protein suspension comprises 10 to 15%, 11 to 15%, 12 to 15%, 13 to 15% or 14 to 15% denatured plant protein (by weight).

In any embodiment, at least 90%, 91%, 92%, 93%, 94% or 95% of the protein in the denatured plant protein suspension is solubilised.

In another aspect, the invention provides a denatured plant protein suspension formed according to the method of the invention.

In another aspect, the invention provides a composition of microparticles in which the microparticles comprise active agent contained within a denatured plant protein structure, in which the denatured plant protein structure typically comprises polymerised denatured plant protein .

In any embodiment, the active agent is contained within a carrier. The carrier may be edible oil droplets, in which the active agent is contained within the edible droplets, or a carrier powder such as native protein or sugar such as maltodextrin or starch.

In any embodiment, the polymerised denatured plant protein structure is a polymerised denatured plant protein matrix, and the oil drops are distributed through the matrix.

In any embodiment, the microparticle has a core-shell structure comprising a denatured plant protein shell and a core comprising the active agent typically in a carrier. The carrier may be a powder (as described above), edible oil droplets, or a solution/suspension of active agent.

In any embodiment, the denatured plant protein shell is polymerised.

In any embodiment, the microparticle has two or more shells in which at least one shell is a denatured plant protein shell and at least one shell is selected from an oil shell, and a non-polymerised denatured plant protein shell.

In any embodiment, the microparticle is a granule.

In another aspect, the invention provides a food, beverage or nutritional supplement, in any form, comprising a composition of the invention.

In any embodiment, the beverage, food or nutritional supplement is shelf stable.

In any embodiment, the food or beverage is pasteurised or UHT treated.

In any embodiment, the beverage is a carbonated or non-carbonated acidic beverage.

In any embodiment, the beverage is a fermented beverage (e.g. kombucha).

In any embodiment, the method has a % probiotic survival of greater than 60%, 65%, 70%, 75%, 80%, 85% or 90%.

In any embodiment, the agglomerated microparticles are gastric resistant and ileal sensitive. This means that the microparticles can pass through a human stomach intact and break up in the ileum releasing the probiotic payload.

Other aspects and preferred embodiments of the invention are defined and described in the other claims set out below.

### Brief Description of the Figures

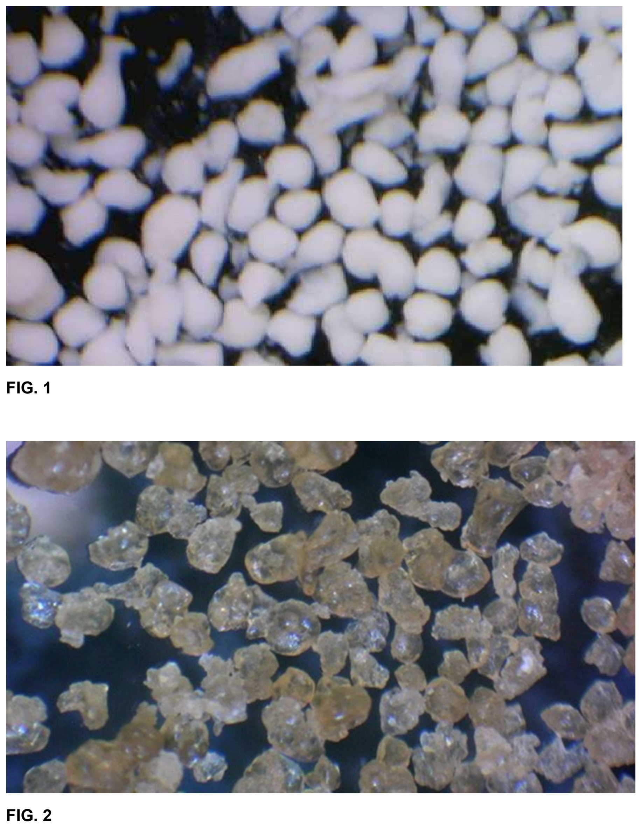

**FIG. 1****:** An image of probiotic-denatured pea protein microcapsules formed according to the method of Example 20 and imaged before the drying stage. This microscope image represents polymerised wet capsules. Average diameter is 500 - 600 um.
**FIG. 2****:** An image of probiotic-denatured pea protein microcapsules formed according to the method of Example 20 and imaged after the drying stage. Microscope image illustrates individual microcapsule with free-flowing format. Average diameter 485 - 550 um.

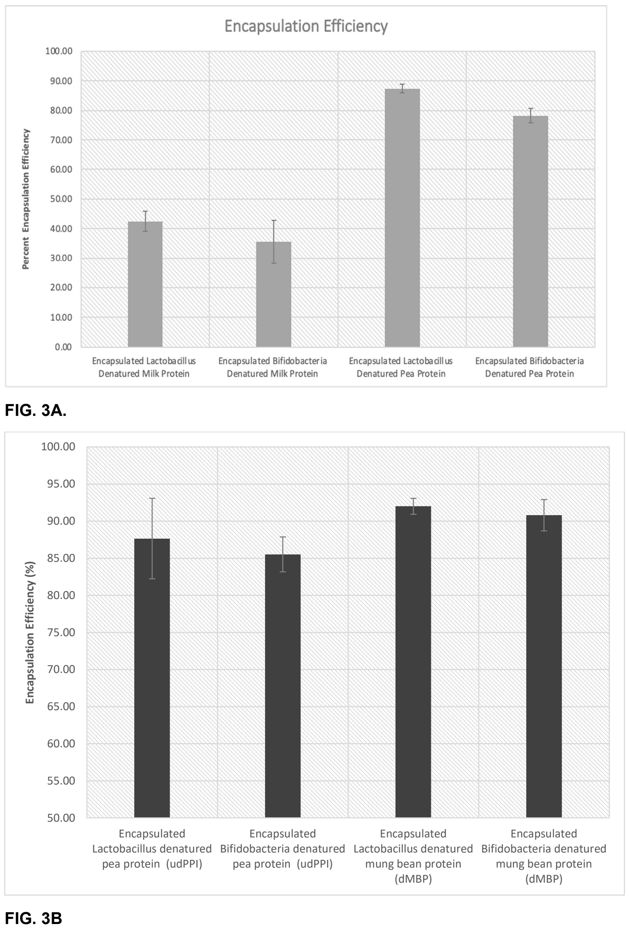

**FIG. 3A****:** Summary of percentage probiotic survival of probiotic encapsulation using pea protein vs whey (milk) protein and a single nozzle encapsulator.
**FIG. 3B****:** Summary of percentage probiotic survival of probiotic encapsulation using various plant protein encapsulation matrices and a single nozzle extrusion technique.

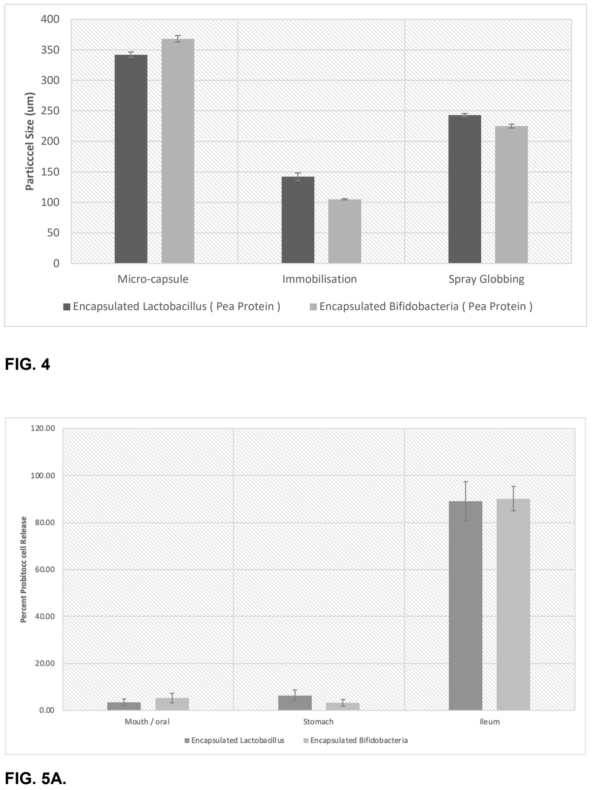

**FIG. 4****:** Comparison of particle size of probiotic encapsulation systems made using pea protein and a single nozzle encapsulator.
**FIG. 5A****:** Percentage probiotic release profile of encapsulated probiotic cells in a pea protein encapsulation system made using single nozzle encapsulation extrusion technique. Data represents cell liberation as a function of in vitro human digestion time and GI section.

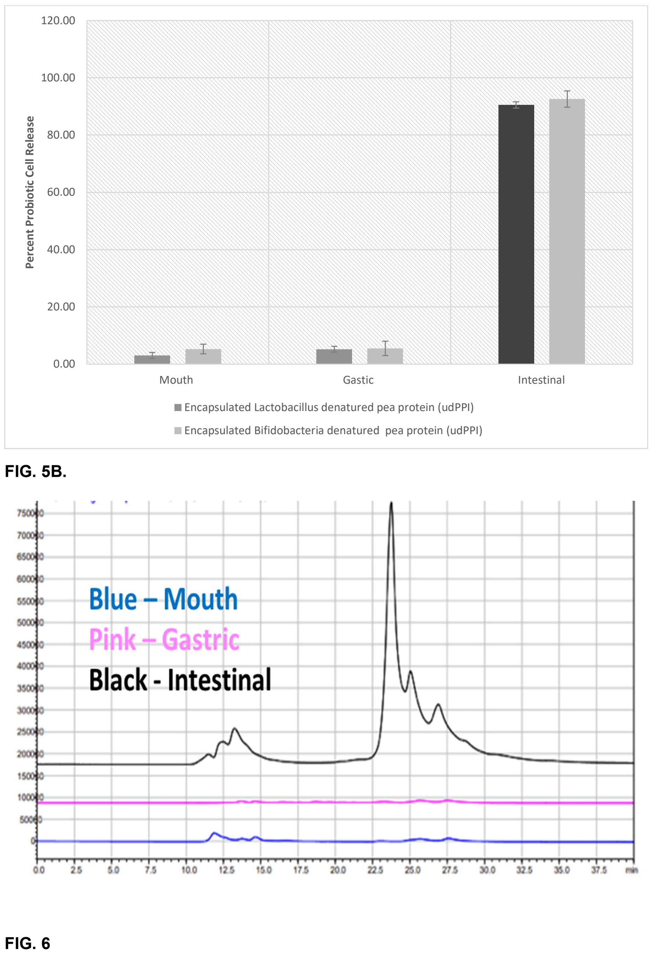

**FIG. 5B****:** Percentage probiotic release profile of encapsulated probiotic cells in an ultra-denatured pea protein encapsulation system produced using single nozzle encapsulation / extrusion technique. Data represents cell liberation as a function of in vitro human digestion time and GI section.
**FIG. 6****:** HPLC protein digestion as a function of GI section. The HPLC data illustrates start of protein breakdown in the intestine, which indicates controlled cell release. The data represent the digestion profile for cell liberation from a pea protein encapsulation system made using extrusion, single nozzle encapsulation technique.

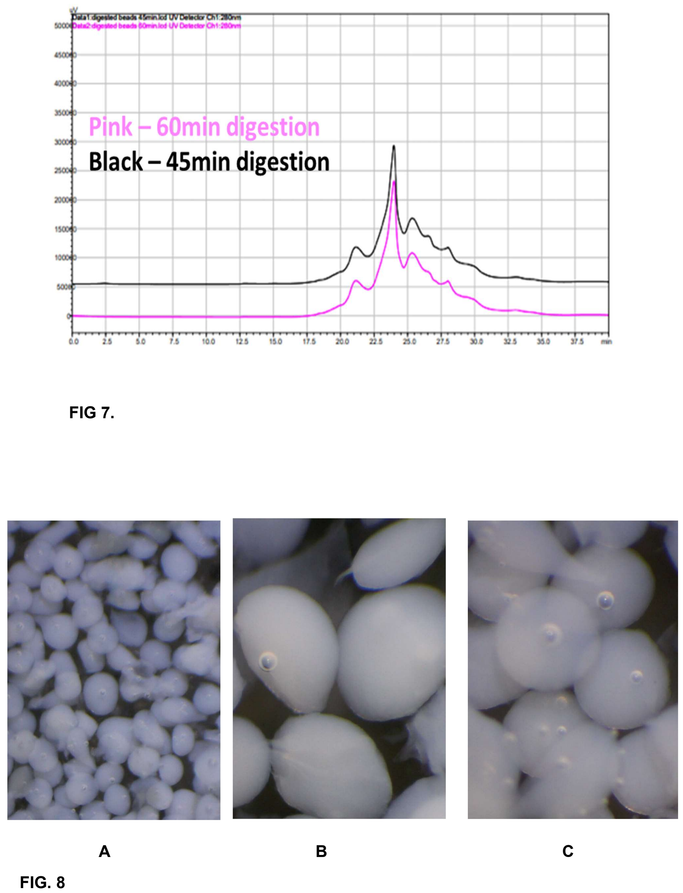

**FIG. 7****:** HPLC chromatogram for quantification of protein break-down during human in vitro digestion analysis. Data illustrates the initiation of protein digestion in the intestine after 45 minutes (black) and 60 minutes (pink), which indicates controlled cell release due to the sustained and controlled digestion of the plant protein microcapsule. The data represents the digestion profile for cell liberation from a plant protein encapsulation system made using extrusion, single nozzle encapsulation technique.
**FIG. 8****:** Microscope images of bifidobacteria (lactic acid bacteria) encapsulated in plant proteins: (A) Mung bean protein, (B) Fava bean protein, and (C) Chickpea protein generated by single nozzle / extrusion technique.

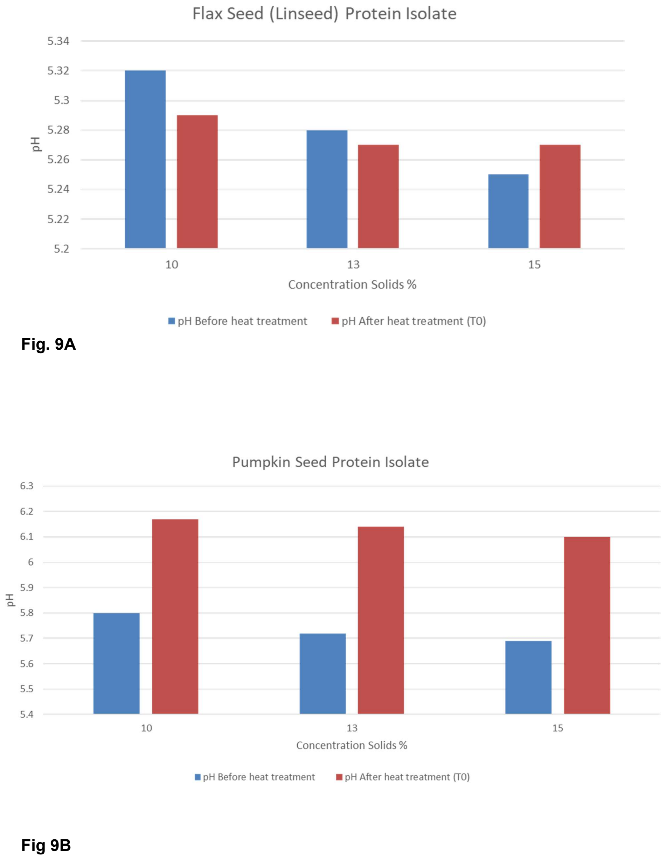

**FIG. 9****:** pH characteristics of plant proteins before and after UHT plant protein denaturation (A) Flax seed (Linseed) protein isolate and (B) Pumpkin seed Protein isolate.

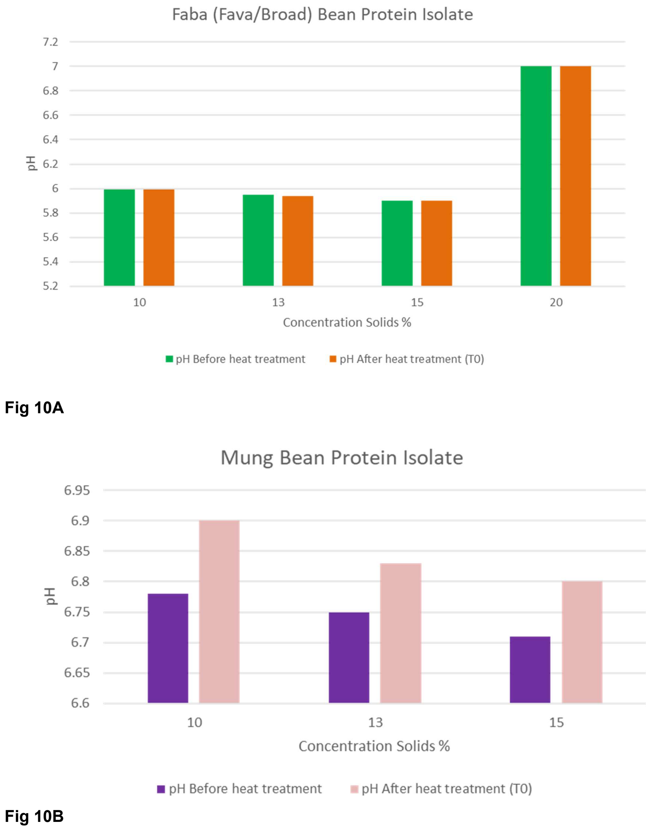

**FIG. 10****:** pH characteristics of plant proteins before and after UHT plant protein denaturation. (A) Fava (Fava/Broad) bean protein isolate and (B) Mung bean protein isolate.

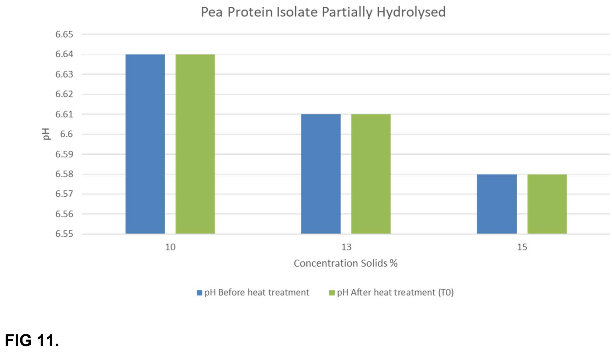

**FIG. 11****:** pH characteristics of pea protein hydrolysate before and after UHT pea protein denaturation procedure. This verifies denaturation of pea protein materials that are partially hydrolysed.

### Detailed Description of the Invention

All publications, patents, patent applications and other references mentioned herein are hereby incorporated by reference in their entireties for all purposes as if each individual publication, patent or patent application were specifically and individually indicated to be incorporated by reference and the content thereof recited in full.

Where used herein and unless specifically indicated otherwise, the following terms are intended to have the following meanings in addition to any broader (or narrower) meanings the terms might enjoy in the art:
Unless otherwise required by context, the use herein of the singular is to be read to include the plural and *vice versa.* The term "a" or "an" used in relation to an entity is to be read to refer to one or more of that entity. As such, the terms "a" (or "an"), "one or more," and "at least one" are used interchangeably herein.

As used herein, the term "comprise," or variations thereof such as "comprises" or "comprising," are to be read to indicate the inclusion of any recited integer (e.g. a feature, element, characteristic, property, method/process step or limitation) or group of integers (e.g. features, element, characteristics, properties, method/process steps or limitations) but not the exclusion of any other integer or group of integers. Thus, as used herein the term "comprising" is inclusive or openended and does not exclude additional, unrecited integers or method/process steps.

As used herein, the term "plant protein" refers to a protein preparation obtained from a plant source. Examples include plant protein powders, plant protein concentrates and plant protein isolates. Plant protein preparations can be obtained from pea, zein, barley, spelt, soya, seaweed, hemp, seiten (wheat gluten), chickpea, tofu, lentil, mung bean, quinoa and other plant sources. Plant proteins having an isoelectric point above pH 5, 5.5 or 6 are preferred.

As used herein, the term "simple carbohydrate" includes monosaccharides such as glucose, fructose and sucrose and disaccharides such as sucrose, lactose and maltose, a mixtures thereof. In one embodiment, the simple carbohydrate comprises GLUCIDEX.

As used herein, the term "probiotic" refers to live beneficial bacteria and/or yeasts that naturally live in your body. Probiotics help keep your body healthy and working well, including fighting off bad bacteria when you have too much of it, helping you feel better. The probiotics employed in the invention may comprise a combination of different probiotics. Though there are many types of bacteria that can be considered probiotics, there are two specific types of bacteria that are common probiotics found in stores. These include Lactobacillus and Bifidobacterium.

As used herein, the term "% probiotic survival" as applied to the compositions made according to the method of the invention means the % of live cells that survive the encapsulation process (including the drying process where the process includes a drying step). % probiotic survival can be measured by determining the live cell input into the process and comparing with the number of cells in the output product.

As used herein, the term "active agent" refers to any component suitable for delivery to the mammalian small intestine or ileum, but typically means a component that is sensitive to an external condition for example heat, pH, moisture, pressure, chemical stress or enzymes. Thus, the active component may be sensitive to pH, enzymes (i.e. protease enzymes), high pressure, moisture, high shear, and temperature abuse during storage. In one particularly preferred embodiment of the invention, the active agent comprises a probiotic or a bioactive agent selected from, for example, caffeine, fat soluble vitamins (A, D, E, K), water soluble vitamins (B and C), hyaluronic acid, melatonin, and marine bioactives such as fucoidan and laminarin, green, brown and red seaweed extract.

"Gastric-resistant": means that the microparticles can survive intact for at least 60 minutes in the simulated stomach digestion model described in Minekus et al., 1999 and 2014 (A computer-controlled system to simulate conditions of the large intestine with peristaltic mixing, water absorption and absorption of fermentation product, Minekus, M., Smeets-Peeters M, Bernalier A, Marol-Bonnin S, Havenaar R, Marteau P, Alric M, Fonty G, Huis in't Veld JH, Applied Microbiology Biotechnology. 1999 Dec;53 (1):108-14) and (Minekus et al., 2014, A standardised static in vitro digestion method suitable for food - an international consensus, Minekus, A. et al., Food Function, 2014, 5, 1113).

"Ileal-sensitive": means that the microparticles are capable of releasing their contents in vivo in the ileum (proximal or distal) of a mammal.

### Exemplification

The invention will now be described with reference to specific Examples. These are merely exemplary and for illustrative purposes only: they are not intended to be limiting in any way to the scope of the monopoly claimed or to the invention described. These examples constitute the best mode currently contemplated for practicing the invention.

### EXAMPLE 1

### Preparation of Pea Protein Solution for Denaturation

- Hydrate 14 % (w/v) pea protein isolate in water or phosphate buffer 0.1M pH 7.5
- Hydrate at 30 °C for 45 minutes
- Agitate at 450 RPM using jacketed vessel or hot plate at 30 °C
- Leave for minimum 90 min as the request resting step
- Check pH pf water and verify pH 7.5 - if not, adjust using 1 N HCl / NaOH
- Heat treat as follows
   - Pre heat 65 °C for a minimum of 2 seconds/ maximum of 15 minutes
   - Final heat 95.5 °C for a minimum of 2 seconds/ maximum of 3 minutes
   - Cooling temperature 22-25 °C
   - Flow Rate 1.5 L - 20 L per minute
   - Pressure 1.3 - 1.6 bar
- Allow material to settle for 4 h at room temperature and it is ready for use
- This material is referred to as "dPPI".

### EXAMPLE 2

### Preparation of Ultra - Denatured Pea Protein

- Hydrate 14 % (w/v) pea protein isolate in water or phosphate buffer 0.1 M pH 7.5
- Hydrate at 30 °C for 45 minutes
- Agitate at 450 RPM using jacketed vessel
- Leave for minimum 90 min as the request resting step
- Check pH pf water and verify pH 7.5 - if not, adjust using 1 N HCl / NaOH
- Heat treat as follows:
   Pre heat 60- 98 °C for min 2 seconds
   Final heat 126 - 144.5 °C
   Hold time - minimum 2 seconds for each step a
   Cooling temperature 22-25 °C
   Flow Rate: min 1.45 L - 2.0 L per minute
   Pressure 1.0 - 6.0 bar
- Allow material to settle for 2 hour at room temperature and it is ready for use
- This material is referred to as "udPPI"

### EXAMPLE 3

### Preparation of Ultra - Denatured mung bean Protein

- Hydrate 10 % (w/v) mung protein isolate in water or phosphate buffer 0.1M pH7.5
- Hydrate at 30 °C for 90 minutes
- Agitate at 450 RPM using jacketed vessel
- Leave for minimum 90 min as the request resting step
- Check pH pf water and verify pH 7.5 - if not, adjust using 1 N HCl / NaOH
- Heat treat as follows:
   Pre heat 60- 90 °C
   Final heat 120 - 144 °C for min 2 seconds
   Cooling temperature 22- 25 °C for min 5 seconds
   Flow Rate 1.5 L - 2.0 L per minute
   Pressure 1.0 - 6.0 bar
- Allow material to settle for 2 hour at room temperature and it is ready for use
- This material is referred to as "udMBP".

### EXAMPLE 4

### Hydration of probiotic

- Make stock solution of probiotic - hydrate in phosphate buffer pH 0.1 M
- Add probiotic (liquid or powder form) to phosphate buffer
- Agitate at 200 - 450 RPM for 10 - 45 minutes at RT for resuscitation of the cells
- Ultraturrax cell suspension if required (9,000 - 11,000 RPM for 30 - 90 seconds)

### EXAMPLE 5

### Immobilisation of probiotics in PPI using denatured PPI

- Using dPPI prepared as per Example 1 and hydrated probiotic prepared as per Example 4, add probiotic suspension to dPPI solution at 410RPM to provide a suspension of PPI and probiotic in 9:1 or 18:1 ratio.
- Add 2% glucidex / simple carbohydrate to the protein solution prior to encapsulation (this adds charge to the denatured protein solution the create a compatible matrix for probiotics)
- Hydrate at RT for 2 hours using mild agitation to avoid creation of air pockets
- Once all probiotic is added, after 2 minutes add 0.1 M/ pH 6.1 Calcium Citrate or Calcium Chloride
- Add the Calcium Citrate until the buffer / suspension ratio is 1:200.
- During pH adjustment, maintain high level of agitation at 450 RPM RT
- Maintain agitation for 15 minutes minimum and transfer to drying chamber
- The solution will visibly appear more viscous.
- Drying (Freeze-drying or Vacuum drying) can be conducted as follows:
- Freeze Drying (See Table 1)

**Table 1: Two Freeze Drying Runs (48 and 72 hour runs)**

| - **Temperature** | **Time** | **Temperature** | **Time** |
|---|---|---|---|
| -25°C | 23 Hours | -25°C | 18 Hours |
| -10°C | 25 Hours | -10°C | 12 Hours |
| 0°C | 8 Hours | 0°C | 2 Hours |
| 10°C | 8 Hours | 10°C | 2 Hours |
| 20°C | 4 Hours | 25°C | 2 Hours |
| 25°C | 4 Hours | | |
| **Total Drying Time = 72 Hours** | | **Total Drying Time = 48 Hours** | |

- Vacuum Drying:
Set product temperature to 25 °C
Create vacuum in product chamber
Dry for 24 h once full vacuum is achieved i.e. < 10mBar

After drying, the dry product can be size reduced into a particulate form using a high shear solid separator.

### EXAMPLE 6

### Immobilisation of probiotics in plant protein using denatured mung bean

- Using dMBP prepared as per Example 3 and hydrated probiotic prepared as per Example 4, add probiotic suspension to dMBP solution at 410RPM to provide a suspension of dMBP and probiotic in 9:1 or 18:1 ratio.
- Add 2% glucidex / simple carbohydrate to the protein solution prior to encapsulation (this adds charge to the denatured protein solution the create a compatible matrix for probiotics )
- Hydrate at RT for 2 hours using mild agitation to avoid creation of air pockets
- Once all probiotic is added, after 2 minutes add 0.1 M/ pH 6.1 Calcium Citrate or Calcium Chloride
- Add the Calcium Citrate until the buffer / suspension ratio is 1:200.
- During pH adjustment, maintain high level of agitation at 450 RPM RT
- Maintain agitation for 15 minutes minimum and transfer to drying chamber
- The solution will visibly appear more viscous.
- Drying (Freeze-drying or Vacuum drying) can be conducted as follows:
- Freeze Drying (See Table 1)
- Vacuum Drying:
   Set product temperature to 25 °C
   Create vacuum in product chamber
   Dry for 24 h once full vacuum is achieved i.e. < 10mBar

After drying, the dry product can be size reduced into a particulate form using a high shear solid separator.

### EXAMPLE 7

### Microbeads comprising probiotic encapsulated in denatured mung bean protein - single nozzle extrusion

- Using dMBP prepared as per Example 3, prepare a suspension of dMBP and agitate at 250 RPM at RT
- Add 2% glucidex / simple carbohydrate to the protein solution prior to encapsulation (this adds charge to the denatured protein solution and creates a compatible matrix for probiotics)
- Hydrate at room temperature for 2 hours using mild agitation to avoid creation of air pockets
- Add the hydrated probiotic suspension of Example 4 to dMBP solution, that is agitating at RT
- Add probiotic suspension to protein at 9:1 or 18:1 (protein: probiotic ratio)
- Agitate the Protein - Probiotic Suspension at 400 - 450 RPM for 10 minutes

### PREPARATION OF CURING BATH

- Prepare Calcium citrate buffer 0.1 M; adjust pH to 5.2 (range is 5.2 - 6.1). Add 0.05% Polysorbate 20% as a surfactant
- Alternative curing bath is Calcium Chloride buffer 0.1 M; adjust pH to 4.8 (range is 4.3 - 5.0). Add 0.05% Polysorbate 20% as a surfactant
- Alternative curing bath is Calcium Citrate buffer 0.1 M; with 0.05% chitosan calcium chelator) and 0.05% Polysorbate 20% (surfactant)
- Once probiotic is fully hydrated, extrude the protein- probiotic solution through a single nozzle extruder at Room Temperature for polymerisation in the calcium curing bath
- Polymerisation will occur in the calcium buffer bath at Room Temperature
- Microcapsules are collected and washed in neutralising solution at pH 6.1 (prevents sticking of microcapsules during drying - creating optimised yield)
- Micro-capsules are loaded to the relevant drying chamber
- Drying can be conducted as follows:
   ∘ Freeze Drying for 48 h (See Table 1)
   ∘ Fluidised Bed Drying: 37 °C for 4-6 Hours
   ∘ Vacuum Drying: Less than 10 mBar for 24 - 48 hours minimum at 37 °C

### EXAMPLE 8

### Microbeads comprising probiotic encapsulated in denatured plant protein - single nozzle extrusion

- Using denatured plant protein prepared as per Example 1, prepare a suspension of denatured protein and agitate at 250 RPM at RT
- Add 2% - 4% simple carbohydrate to the protein solution prior to encapsulation (this adds charge to the denatured protein solution and creates a compatible matrix for probiotics)
- Hydrate at room temperature for 2 hours using mild agitation to avoid creation of air pockets
- Add the hydrated probiotic suspension of denatured protein solution, that is agitating at RT
- Add probiotic suspension to protein at 9:1 or 18:1 (protein: probiotic ratio)
- Agitate the Protein - Probiotic Suspension at 400 - 450 RPM for 10 minutes

### PREPARATION OF CURING BATH

- Prepare Calcium citrate buffer 0.1M; adjust pH to 5.2 (range is 5.2 - 6.1). Add 0.05% Polysorbate 20% as a surfactant
- Alternative curing bath is Calcium cation buffer 0.1 M; adjust pH to 4.8 (range is 4.3 - 5.0). Add 0.05% Polysorbate 20% as a surfactant
- Alternative curing bath is Calcium Citrate buffer 0.1 M; with 0.05% chitosan calcium chelator) and 0.05% Polysorbate 20% (surfactant)
- Once probiotic is fully hydrated, extrude the protein-probiotic solution through a single nozzle extruder at Room Temperature for polymerisation in the calcium curing bath.
- Polymerisation will occur in the calcium buffer bath at Room Temperature
- Microcapsules are collected and washed in neutralising solution at pH 6.1 (prevents sticking of micro- capsules during drying - creating optimised yield
- Micro-capsules are loaded to the relevant drying chamber
- Drying can be conducted as follows:
   ∘ Freeze Drying for 48 hours (See Table 1)
   ∘ Fluidised Bed Drying: 37 °C for 4-6 Hours
   ∘ Vacuum Drying: Less than 10mBar for 24 - 48 hours min at 37 °C

### EXAMPLE 9

### Microbeads comprising probiotic encapsulated in denatured mung bean protein - double (concentric) nozzle extrusion

- Using dMBP prepared as per Example 3, prepare a suspension of dMBP agitate at 250 RPM at RT.
- Add 2% glucidex / simple carbohydrate to the protein solution prior to encapsulation (this adds charge to the denatured protein solution and creates a compatible matrix for probiotics)
- Hydrate at room temperature for 2 hours using mild agitation to avoid creation of air pockets
   ∘ Prepare a calcium bath according to Example 7.
- Simultaneously extrude the suspension of dMBP and the hydrated probiotic through the concentric nozzle into the curing bath, with the dMBP/glucidex suspension extruded through the outer nozzle and the probiotic suspension extruded through the inner nozzle
- Polymerisation will occur in the calcium buffer bath at Room Temperature
- Micro-capsules are collected and washed in neutralising solution at pH 6.1 (prevents sticking of micro- capsules during drying - creating optimised yield
- Micro-capsules are loaded to the relevant drying chamber
- Drying can be conducted as follows:
   ∘ Freeze Drying for 48 h (See Table 1)
   ∘ Fluidised Bed Drying: 37 °C for 4-6 Hours
   ∘ Vacuum Drying: Less than 10 mBar for 24 - 48 hours min at 37 °C

### EXAMPLE 10

### Microbeads comprising probiotic encapsulated in denatured plant protein - double (concentric) nozzle extrusion

- Using denatured plant protein prepared as per Example 1, prepare a suspension of dPP agitate at 250 RPM at RT.
- Add 2% - 4% glucidex / simple carbohydrate to the protein solution prior to encapsulation (this adds charge to the denatured protein solution and creates a compatible matrix for probiotics)
- Hydrate at room temperature for 2 hours using mild agitation to avoid creation of air pockets
   ∘ Prepare a calcium bath according to Example 7.
- Simultaneously extrude the suspension of dPP and the hydrated probiotic through the concentric nozzle into the curing bath, with the dPP/glucidex suspension extruded through the outer nozzle and the probiotic suspension extruded through the inner nozzle
- Polymerisation will occur in the calcium buffer bath at Room Temperature
- Micro- capsules are collected and washed in neutralising solution at pH 6.1 (prevents sticking of micro- capsules during drying - creating optimised yield
- Micro-capsules are loaded to the relevant drying chamber
- Drying can be conducted as follows:
   ∘ Freeze Drying for 48 h (See Table 1)
   ∘ Fluidised Bed Drying: 37 °C for 4- 6 Hours
   ∘ Vacuum Drying: Less than 10mBar for 24 - 48 hours min at 37 °C

### EXAMPLE 11

### Probiotic Spray Englobing using denatured pea protein dPPI

- Prepare dry ingredients and load into chamber (protein, probiotic and simple sugar)
- Prepare denatured protein suspension
- Begin fluidisation process and activate heating (35 - 45 °C)
- Fluidise with an airflow of 110 - 300 m³/hr with set air inlet temperatures
- Once product temperature is equilibrated, commence spray englobing process using denatured protein
- Nozzle Pressure should be maintained between 2 - 4.5 Bar nozzle pressure air flow adjusted accordingly
- Apply interim drying step to reduce moisture by 50%
- Re-commence spray englobing process using maltodextrin (15% w/v solution)
- Apply interim drying step to reduce moisture by 25%
- Re-commence spray englobing process using denatured protein and conduct 2 - 3 cycles of spray englobing using denatured protein and the simple carbohydrate solution (maltodextrin)
- Implement a final post dry step to generate final moisture content below 5%

### EXAMPLE 12

### Probiotic Spray Englobing using denatured pea protein dPPI and calcium cation cross-linker

- Prepare dry ingredients (protein, probiotic and simple sugar )and load into chamber
- Prepare denatured protein suspension and calcium cation cross-linker
- Begin fluidisation process and activate heating (35 - 45 °C)
- Fluidise with an airflow of 110 - 300 m³/hr with set air inlet temperatures
- Once product temperature is equilibrated, commence spray englobing process using denatured protein
- Nozzle Pressure should be maintained between 2 - 4.5 Bar nozzle pressure air flow adjusted accordingly
- Apply interim drying step to reduce moisture by 50%
- Re-commence spray englobing process using Calcium chloride (0.2 - 0.4 M)
- Apply interim drying step to reduce moisture by 25%
- Re-commence spray englobing process using denatured protein and conduct 2 - 3 cycles of spray englobing using denatured protein and the calcium cross-linker
- Implement a final post dry step to generate final moisture content below 5%

### EXAMPLE 13

### Probiotic Spray Englobing using denatured pea protein, high oleic coconut oil and calcium cation cross-linker

- Prepare dry ingredients (protein, probiotic and simple sugar) and load into chamber
- Prepare denatured pea protein suspension and calcium cation cross-linker
- Begin fluidisation process and activate heating (35 - 45 °C)
- Fluidise with an airflow of 110 - 300 m³/hr with set air inlet temperatures
- Once product temperature is equilibrated, commence spray englobing process using denatured protein
- Nozzle Pressure should be maintained between 2 - 4.5 Bar nozzle pressure air flow adjusted accordingly
- Apply interim drying step to reduce moisture by 50%
- Re-commence spray englobing process using high oleic coconut oil
- Apply interim drying step to reduce moisture by 25%
- Re-commence spray englobing process using Calcium chloride (0.2 - 0.4 M)
- Apply interim drying step to reduce moisture by 25%
- Re-commence spray englobing process using denatured protein and conduct 2 - 3 cycles of spray englobing using denatured protein and the calcium or magnesium cation cross-linker
- Implement a final post dry step to generate final moisture content below 5%

### EXAMPLE 14

### Probiotic Spray Englobing using denatured mung protein and calcium cation cross-linker

- Prepare dry ingredients (protein, probiotic and simple sugar) and load into chamber
- Prepare denatured mung protein suspension and calcium cation cross-linker
- Begin fluidisation process and activate heating (35 - 45 °C)
- Fluidise with an airflow of 110 - 300 m³/hr with set air inlet temperatures
- Once product temperature is equilibrated, commence spray englobing process using denatured protein
- Nozzle Pressure should be maintained between 2 - 4.5 Bar nozzle pressure air flow adjusted accordingly
- Apply interim drying step to reduce moisture by 50%
- Re-commence spray englobing process using Calcium chloride (0.2- 0.4 M)
- Apply interim drying step to reduce moisture by 25%
- Re-commence spray englobing process using denatured protein and conduct 2 - 3 cycles of spray englobing using denatured protein and the calcium or magnesium cation cross-linker
- Implement a final post dry step to generate final moisture content below 5%

### EXAMPLE 15

### Probiotic Spray Englobing using denatured mung protein, high oleic sunflower oil and calcium cation cross-linker

- Prepare dry ingredients (protein, probiotic and simple sugar) and load into chamber
- Prepare denatured mung protein suspension and calcium cation cross-linker
- Begin fluidisation process and activate heating (35 - 45 °C)
- Fluidise with an airflow of 110 - 300 m³/hr with set air inlet temperatures
- Once product temperature is equilibrated, commence spray englobing process using denatured plant protein
- Nozzle Pressure should be maintained between 2 - 4.5 Bar nozzle pressure air flow adjusted accordingly
- Apply interim drying step to reduce moisture by 50%
- Re-commence spray englobing process using high oleic sunflower oil
- Apply interim drying step to reduce moisture by 25%
- Re-commence spray englobing process using Calcium chloride (0.2 - 0.4 M)
- Apply interim drying step to reduce moisture by 25%
- Re-commence spray englobing process using denatured protein and conduct 2 - 3 cycles of spray englobing using denatured protein and the calcium or magnesium cation cross-linker
- Implement a final post dry step to generate final moisture content below 5%

### EXAMPLE 16

### Probiotic Spray Englobing using denatured mung protein, high oleic coconut oil and calcium cation cross-linker

- Prepare dry ingredients (protein, probiotic and simple sugar) and load into chamber
- Prepare denatured mung protein suspension and calcium cation cross-linker
- Begin fluidisation process and activate heating (35 - 45 °C)
- Fluidise with an airflow of 110 - 300 m³/hr with set air inlet temperatures
- Once product temperature is equilibrated, commence spray englobing process using denatured protein
- Nozzle Pressure should be maintained between 2 - 4.5 Bar nozzle pressure air flow adjusted accordingly
- Apply interim drying step to reduce moisture by 50%
- Re-commence spray englobing process using high oleic coconut oil
- Apply interim drying step to reduce moisture by 25%
- Re-commence spray englobing process using high oleic sunflower oil
- Apply interim drying step to reduce moisture by 25%
- Re-commence spray englobing process using Calcium chloride (0.2 - 0.4 M)
- Apply interim drying step to reduce moisture by 25%
- Re-commence spray englobing process using denatured protein and conduct 2 - 3 cycles of spray englobing using denatured protein and the calcium cation cross-linker
- Implement a final post dry step to generate final moisture content below 5%

### EXAMPLE 17

### Probiotic Spray Englobing using denatured pea protein, high oleic coconut oil

- Prepare dry ingredients (protein, probiotic) and load into chamber
- Prepare denatured mung protein suspension and calcium cation cross-linker
- Begin fluidisation process and activate heating (35 - 45 °C)
- Fluidise with an airflow of 110 - 300 m³/hr with set air inlet temperatures
- Once product temperature is equilibrated, commence spray englobing process using denatured protein
- Nozzle Pressure should be maintained between 2 - 4.5 Bar nozzle pressure air flow adjusted accordingly
- Apply interim drying step to reduce moisture by 50%
- Re-commence spray englobing process using high oleic coconut oil
- Apply interim drying step to reduce moisture by 25%
- Re-commence spray englobing process using simple carbohydrate (maltodextrin)
- Apply interim drying step to reduce moisture by 25%
- Re-commence spray englobing process using denatured protein and conduct 2 - 3 cycles of spray englobing using denatured protein and the simple carbohydrate solution
- Implement a final post dry step to generate final moisture content below 5%

### EXAMPLE 18

### Probiotic Spray Englobing using denatured mung protein and calcium cation cross-linker with optimised coating layer

- Prepare dry ingredients (protein, probiotic) and load into chamber
- Prepare denatured mung protein suspension and calcium cation cross-linker
- Begin top -spray fluidisation process and activate heating (35 - 45 °C)
- Fluidise with an airflow of 110 - 300 m³/hr with set air inlet temperatures
- Once product temperature is equilibrated, commence spray englobing process using denatured protein
- Nozzle Pressure should be maintained between 2 - 4.5 Bar nozzle pressure air flow adjusted accordingly
- Apply interim drying step to reduce moisture by 50%
- Re-commence spray englobing process using Calcium chloride (0.2 - 0.4M)
- Apply interim drying step to reduce moisture by 25%
- Re-commence spray englobing process using denatured protein and conduct 2 - 3 cycles of spray englobing using denatured protein and the magnesium cation cross-linker
- Implement a final post dry step to generate final moisture content below 5%
- Initiate bottom spray englobing using denatured mung bean protein
- Utilised nozzle pressure in excess of 2 Bar
- Maintain accelerator air pressure above 1.5 Bar during spray englobing
- Maintain airflow at equilibration (approx 90 - 170 m³/hr)
- Maintain product temperature between 37 - 45 °C
- Apply intermittent post drying at 45 - 55 °C
- Implement at least 2 cycles of calcium cross-linking spray englobing steps with subsequent intermittent drying to achieve 25% reduction on moisture content
- Apply final post dry step to achieve final moisture content below 5%.

### EXAMPLE 19

### Probiotic Spray Englobing using denatured pea protein, high oleic coconut oil and calcium cation cross-linker with optimised coating layer

- Prepare dry ingredients (protein, probiotic) and load into chamber
- Prepare denatured mung protein suspension and calcium cation cross-linker
- Begin top-spray fluidisation process and activate heating (35 - 45 °C)
- Fluidise with an airflow of 110 - 300 m³/hr with set air inlet temperatures
- Once product temperature is equilibrated, commence spray englobing process using denatured protein
- Nozzle Pressure should be maintained between 2 - 4.5 Bar nozzle pressure air flow adjusted accordingly
- Apply interim drying step to reduce moisture by 50%
- Re-commence spray englobing process using high oleic coconut oil
- Apply interim drying step to reduce moisture by 25%
- Re-commence spray englobing process using Calcium chloride (0.2 - 0.4 M)
- Apply interim drying step to reduce moisture by 25%
- Re-commence spray englobing process using denatured protein and conduct 2 - 3 cycles of spray englobing using denatured protein and the magnesium cation cross-linker
- Implement a final post dry step to generate final moisture content below 5%
- Initiate bottom spray englobing using denatured protein
- Utilised a nozzle pressure in excess of 2 Bar
- Maintain accelerator air pressure above 1.5 Bar during spray englobing
- Maintain airflow at equilibration (approx 90 - 170 m³/hr)
- Maintain product temperature between 37 - 45 °C
- Apply intermittent post drying at 45 - 55 °C
- Apply spray englobing process using high oleic coconut oil
- Apply interim drying step to reduce moisture by 25%
- Implement at least 2 cycles of calcium cross-linking spray englobing steps with subsequent intermittent drying to achieve 25% reduction on moisture content
- Apply final post dry step to achieve final moisture content below 5%

### EXAMPLE 20

### Microbeads comprising probiotic encapsulated in denatured PPI (dPPI)- single nozzle extrusion

- Using dPPI prepared as per Example 1, prepare a suspension of dPPI and agitate at 250 RPM at RT
- Add 2% glucidex / simple carbohydrate to the protein solution prior to encapsulation (this adds charge to the denatured protein solution and creates a compatible matrix for probiotics)
- Hydrate at room temperature for 2 hours using mild agitation to avoid creation of air pockets
- Add the hydrated probiotic suspension of Example 2 to dPPI solution, that is agitating at RT
- Add probiotic suspension to protein at 9:1 or 18:1 (protein: probiotic ratio)
- Agitate the Protein - Probiotic Suspension at 400 - 450 RPM for 10 minutes

### PREPARATION OF CURING BATH

- Prepare Calcium citrate buffer 0.1 M; adjust pH to 5.2 (range is 5.2 - 6.1). Add 0.05 (w/w)% Polysorbate 20% as a surfactant
- Alternative curing bath is Calcium Chloride buffer 0.1M; adjust pH to 4.8 (range is 4.3 - 5.0 ). Add 0.05% Polysorbate 20% as a surfactant
- Alternative curing bath is Calcium Citrate buffer 0.1M; with 0.05% chitosan calcium chelator) and 0.05% (w/aw)Polysorbate 20% (surfactant)
- Once probiotic is fully hydrated, extrude the protein- probiotic solution through a single nozzle extruder at Room Temperature for polymerisation in the calcium curing bath
- Polymerisation will occur in the calcium buffer bath at Room Temperature
- Microcapsules are collected and washed in neutralising solution at pH 6.1 (prevents sticking of micro- capsules during drying - creating optimised yield
- Micro-capsules are loaded to the relevant drying chamber
- Drying can be conducted as follows:
   ∘ Freeze Drying for 48 hours (See Table 1)
   ∘ Fluidised Bed Drying: 37 °C for 4-6 Hours
   ∘ Vacuum Drying: Less than 10mBar for 24 - 48 hours min at 37 °C

### EXAMPLE 21

### Microbeads comprising probiotic encapsulated in denatured PPI - double (concentric) nozzle extrusion

- Using dPPI prepared as per Example 1, prepare a suspension of dPPI agitate at 250 RPM at RT.
- Add 2% glucidex / simple carbohydrate to the protein solution prior to encapsulation (this adds charge to the denatured protein solution and creates a compatible matrix for probiotics)
- Hydrate at room temperature for 2 hours using mild agitation to avoid creation of air pockets
   o Prepare a calcium bath according to Example 7.
- Simultaneously extrude the suspension of dPPI and the hydrated probiotic through the concentric nozzle into the curing bath, with the dPPI/glucidex suspension extruded through the outer nozzle and the probiotic suspension extruded through the inner nozzle
- Polymerisation will occur in the calcium buffer bath at Room Temperature
- Micro- capsules are collected and washed in neutralising solution at pH 6.1 (prevents sticking of micro- capsules during drying - creating optimised yield
- Micro-capsules are loaded to the relevant drying chamber
- Drying can be conducted as follows:
   ∘ Freeze Drying for 48 h (See Table 1)
   ∘ Fluidised Bed Drying: 37 °C for 4- 6 Hours
   ∘ Vacuum Drying: Less than 10 mBar for 24 - 48 hours min at 37 °C

### EXAMPLE 22

### Spray englobing of probiotics, bioactives in denature PPI

- Using dPPI prepared as per Example 1, prepare a suspension of dPPI and agitate at 250 RPM at RT.
- Add 2% glucidex / glucose / simple carbohydrate to the protein solution prior to encapsulation (this adds charge to the denatured protein solution and creates a compatible matrix for probiotics).
- Hydrate at room temperature for 2 hours using mild agitation to avoid creation of air pockets.
- Load fluidised bed chamber with dry pea protein powder (native powder; approx. 2% moisture).
- Fluidise at 37 °C at high airflow
- Added probiotic culture to the powder at 5:1 or 10:1 ratio
- Add 15 Calcium Citrate or Calcium Chloride to the chamber and fluidise
- Spray 10% glucidex solution onto the material at high flow rate (20-30 RPM) high airflow(>150 RPM) and low nozzle pressure (<1.5 bar) promotes agglomeration.
- Spray coconut oil / sunflower oil / high oleic oil onto the powder at low flow rate (18-22 RPM), high airflow(>150 RPM) and high nozzle pressure (> 2.5 bar).
- Spray dPPI - glucidex solution into chamber at high flow rate (25-30 RPM), high airflow(>150 RPM) and high nozzle pressure (> 2.5 bar).
- Post dry the material to achieve Aw < 0.15.

### Equivalents

The foregoing description details presently preferred embodiments of the present invention. Numerous modifications and variations in practice thereof are expected to occur to those skilled in the art upon consideration of these descriptions. Those modifications and variations are intended to be encompassed within the claims appended hereto.

## Claims

1. A method of forming a denatured plant protein suspension comprising the steps of:
hydrating a plant protein in an aqueous solvent at pH 6 to 8 or 7 to 8 with agitation and optional heating;
resting the aqueous suspension of plant protein;
adjusting the pH of the aqueous suspension to 6 to 8 or 7 to 8 if necessary;
heating the rested aqueous suspension to denature the plant protein; and
cooling the heated aqueous suspension.
wherein the step of heating the rested aqueous suspension comprises subjecting the rested aqueous suspension to ultra-heat treatment (UHT).

2. A method according to claim 1, in which the rested aqueous suspension is heated to achieve a heat treatment of F0=3.

3. A method according to claim 1, in which the rested aqueous suspension is heated to about 138.5°C x about 3-4 secs.

4. A method according to claim 1, in which the aqueous suspension of plant protein is rested for at least 30 minutes.

5. A method according to claim 1, in which the hydration step is carried out at a temperature of 30 to 40°C.

6. A method according to claim 1, in which the heating step comprises:
pre-heating the rested aqueous suspension under pressure at a first heating temperature of 60 to 70 °C for a first heating time; and
heating the pre-heated aqueous suspension under pressure at a second heating temperature of 90 to 100 °C for a second heating time.

7. A method according to claim 6, in which the heating step is performed at elevated pressure

8. A method according to claim 1, in which the aqueous solvent has a pH of 7.3 to 7.7.

9. A method according to claim 1, in which the plant protein is a plant protein isolate.

10. A method according to claim 1, in which the plant protein is pea protein or mung bean protein.

11. A method according to claim 1, in which the cooled protein suspension is allowed to settle for at least 1 hour at room temperature.

12. A method according to claim 1, in which the method includes a step of adding a simple carbohydrate to the aqueous suspension.

13. A method according to claim 12, in which sufficient simple carbohydrate is added to the aqueous suspension to provide an aqueous suspension comprising 0.5 to 5.0% simple carbohydrate (w/v).

14. A method according to claim 1, in which the aqueous suspension of plant protein comprises 10 to 15% denatured plant protein (by weight).

15. A method according to claim 1, in which the aqueous suspension of plant protein comprises 13 to 15% denatured plant protein (by weight).
